# EUROPEAN PATENT APPLICATION

(11) **EP 3 925 582 A1**
(43) Date of publication of application: **22.12.2021**
(21) Application number: 20766477.2
(22) Date of filing: 27.02.2020
(51) Int. Cl.: A61F 9/007, A61B 90/20, A61B 3/13

(54) **SURGICAL MICROSCOPE SYSTEM, IMAGE PROCESSING METHOD, PROGRAM, AND IMAGE PROCESSING DEVICE**

(30) Priority: 07.03.2019 JP 2019041366
(71) Applicant: Sony Group Corporation, Tokyo 108-0075 (JP)
(72) Inventor: OOTSUKI, Tomoyuki, Tokyo 108-0075 (JP); SOMA, Yoshio, Tokyo 108-0075 (JP); ENOKI, Junichiro, Tokyo 108-0075 (JP)
(74) Representative: D Young & Co LLP
(86) International application number: PCT/JP2020/007905
(87) International publication number: WO 2020/179588

(57) **Abstract**

A surgical microscope system according to an embodiment of the present technology a microscope optical system, an imaging device, and an image processing device. The imaging device captures an image of a visual field range including an eye to be treated through the microscope optical system. The image processing device magnifies a first region in an image including the eye to be treated based on the image captured by the imaging device and reduces a second region other than the first region in the image to be capable of being displayed in a region other than a magnified region that is the first region magnified in the image.

## Description

### Technical Field

The present technology relates to a surgical microscope system, an image processing method, a program, and an image processing device that can be applied to ophthalmic surgery and the like.

### Background Art

In an ophthalmic microscope described in Patent Literature 1, an observational image obtained by imaging patient's left and right eyes is acquired by a user's operation. A captured image obtained by performing processing such as a change of a display scale factor and image correction on the captured observational image is generated. The user can display the observational image or the captured image at a desired timing by switching between the observational image and the captured image. Accordingly, clear and selective presentation of the observational image and the captured image is achieved with a simple configuration (paragraphs [0059] and [0077] in the specification, Figs. 1 and 6, and the like of Patent Literature 1).

### Citation List

### Patent Literature

Patent Literature 1: Japanese Patent Application Laid-open No. 2017-12431

### Disclosure of Invention

### Technical Problem

As described above, it is desirable to provide a technology enabling an image as a target to be sufficiently grasped in an observation using an ophthalmic microscope or the like.

In view of the above-mentioned circumstances, it is an object of the present technology to provide a surgical microscope system, an image processing method, a program, and an image processing device, by which an image including an eye to be treated can be sufficiently grasped.

### Solution to Problem

In order to accomplish the above-mentioned object, a surgical microscope system according to an embodiment of the present technology includes a microscope optical system, an imaging device, and an image processing device.

The imaging device captures an image of a visual field range including an eye to be treated through the microscope optical system.

The image processing device magnifies a first region in an image including the eye to be treated based on the image captured by the imaging device and reduces a second region other than the first region in the image to be capable of being displayed in a region other than a magnified region that is the first region magnified in the image.

In this surgical microscope system, the visual field range including the eye to be treated is imaged through the microscope optical system. The first region in the captured image is magnified and the second region other than the first region is reduced to be capable of being displayed in the region other than the magnified region that is the magnified first region. With this configuration, the image including the eye to be treated can be sufficiently grasped.

The image may be at least a part of a captured image obtained by imaging the eye to be treated.

The image processing device may set a peripheral region adjacent to a periphery of the first region and a region other than the peripheral region to the second region and reduce the peripheral region.

The image processing device may acquire treatment information including at least one of information regarding the eye to be treated or information regarding a treatment to be performed on the eye as a target and determine the first region on the basis of the treatment information.

The image processing device may acquire the treatment information on the basis of the image.

The image processing device may acquire the treatment information from outside.

The treatment information may include at least one of details of the treatment, an instrument to be used in the treatment, a range to be illuminated, a site of the eye, a lesion, or an outline of the image.

The image processing device may be configured to be capable of receiving an instruction of a user and may be configured to determine the first region on the basis of the instruction of the user.

The image processing device may set a predetermined position in the first region as a reference position and magnify the first region by using the reference position as a reference.

The image processing device may magnify the first region such that a distance between the reference position and each position of the first region is increased.

The image processing device may reduce the second region by using the reference position as the reference.

The image processing device may reduce the second region such that a distance between the reference position and each position of an outermost portion of the image.

The image processing device may set a peripheral region adjacent to a periphery of the first region and a region other than the peripheral region to the second region and reduce the second region such that a distance between the reference position and each position of the region other than the peripheral region is maintained.

The image processing device may acquire treatment information including at least one of information regarding an eye to be treated or information regarding a treatment to be performed on the eye as a target and determine the reference position on the basis of the treatment information.

The image processing device may be configured to be capable of receiving an instruction of a user and may be configured to determine the reference position on the basis of the instruction of the user.

The image processing device may be configured to be capable of generating an auxiliary image representing a state of magnification with respect to the first region and representing a state of reduction with respect to the second region.

The auxiliary image may be an image in which the state of magnification and the state of reduction are represented by using at least one of a grid pattern, a luminance, a saturation, a hue, or a boundary line.

An image processing method according to an embodiment of the present technology is an image processing method to be performed by a computer system including: acquiring an image including an eye to be treated. A first region in the image is magnified and a second region other than the first region in the image is reduced to be capable of being displayed in a region other than a magnified region that is the first region magnified in the image.

A program according to an embodiment of the present technology causes a computer system to execute the following steps.

A step of acquiring an image including an eye to be treated.

A step of magnifying a first region in the image and reducing a second region other than the first region in the image to be capable of being displayed in a region other than a magnified region that is the first region magnified in the image.

An image processing device according to an embodiment of the present technology includes an image acquisition unit and an image processing unit.

The image acquisition unit acquires an image including an eye to be treated.

The image processing unit magnifies a first region in the image and reduces a second region other than the first region in the image to be capable of being displayed in a region other than a magnified region that is the first region magnified in the image.

### Brief Description of Drawings

[Fig. 1] A block diagram showing a configuration example of a surgical microscope system according to a first embodiment of the present technology.
[Fig. 2] A block diagram showing a configuration example of an image processing device.
[Fig. 3] A flowchart showing a generation example of a magnified and reduced image.
[Fig. 4] A schematic diagram for describing generation of the magnified and reduced image.
[Fig. 5] A diagram for describing an example of a magnification method for a region to be magnified and a reduction method for a region to be reduced.
[Fig. 6] A graph for describing a setting example of a scale factor at each position.
[Fig. 7] A flowchart showing a specific example of processing of generating the magnified and reduced image.
[Fig. 8] A schematic diagram showing a change in scale factor of a target image obtained by imaging an eye to be treated.
[Fig. 9] A schematic diagram showing an example of detection of a reference position at the time of laser coagulation.
[Fig. 10] A schematic diagram showing an example of detection of a reference position at the time of removing a vitreous body.
[Fig. 11] A schematic diagram showing an example of detection of a reference position at the time of inner limiting membrane peeling.
[Fig. 12] A schematic diagram showing an example of detection of a reference position at the time of drainage of subretinal fluid.
[Fig. 13] A schematic diagram showing an example of detection of a reference position at the time of removing a proliferative membrane.
[Fig. 14] A flowchart showing processing of a presentation image related to a scale factor at each position of a magnified and reduced image according to a second embodiment of the present technology.
[Fig. 15] A schematic diagram showing an example of an auxiliary image representing the scale factor at each position of the magnified and reduced image.
[Fig. 16] A schematic diagram showing an example of an auxiliary image representing the scale factor at each position of the magnified and reduced image.
[Fig. 17] A schematic diagram showing an example of an auxiliary image representing the scale factor at each position of the magnified and reduced image.
[Fig. 18] A schematic diagram showing an example of an auxiliary image representing the scale factor at each position of the magnified and reduced image.

### Mode(s) for Carrying Out the Invention

Hereinafter, embodiments according to the present technology will be described with reference to the drawings.

### <First Embodiment>

### [Surgical Microscope System]

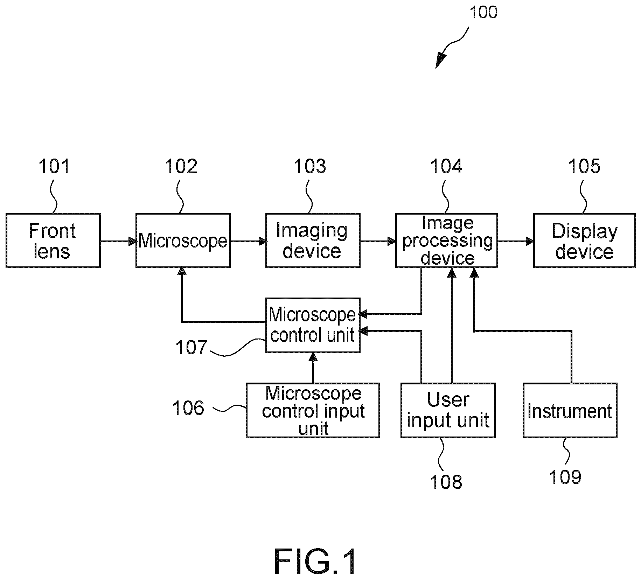

Fig. 1 is a block diagram showing a configuration example of a surgical microscope system 100 according to a first embodiment of the present technology. As shown in the figure, the surgical microscope system 100 includes a front lens 101, a microscope 102, an imaging device 103, an image processing device 104, a display device 105, a microscope control input unit 106, a microscope control unit 107, a user input unit 108, and an instrument 109.

The front lens 101 is capable of being placed at a location between the microscope 102 and a patient's eye. As the front lens 101, a plurality of lenses having different angles of view, such as 90 degrees and 120 degrees, which are suitable for various treatments are selectively used.

The microscope 102 magnifies light emitted from the front lens 101 and causes the light to enter the imaging device 103. The microscope 102 includes a microscope optical system for configuring an optical system microscope. The specific configuration of the microscope 102 is not limited, and may be arbitrarily designed.

The imaging device 103 is mounted on the microscope 102, and captures an image of an eye to be treated through the front lens 101 and the microscope 102. That is, the imaging device 103 can image the field of view of the microscope optical system.

In this embodiment, the imaging device 103 generates a captured image obtained by imaging the eye to be treated. The specific configuration of the imaging device 103 is not limited, and for example, a digital camera including an image sensor such as a complementary metal-oxide semiconductor (CMOS) sensor and a charge coupled device (CCD) sensor is used. Alternatively, for example, an infrared camera equipped with an infrared illumination such as an infrared LED may be used. The imaging device 103 outputs the captured image to the image processing device 104.

The image processing device 104 is called a camera control unit (CCU) and is capable of performing various types of image processing on the captured image output from the imaging device 103. In this embodiment, the image processing device 104 generates a magnified and reduced image, which will be described later in detail. Moreover, the specific details of the image processing that can be performed by the image processing device 104 is not limited, and may be arbitrarily set. For example, in a case where the image of the eye to be treated is inverted through the front lens 101, the image processing device 104 may generate and output inversion-related information about whether or not the inversion is done, the range of the inversion region, and the like.

The display device 105 is capable of displaying various images on the basis of an image (image information) output from the image processing device 104. For example, the magnified and reduced image described above is displayed by the display device 105. Moreover, various graphical user interfaces (GUIs) and the like for performing a treatment, information about the eye to be treated, and the like may be displayed.

The display device 105 is constituted by, for example, a generally-used display or a head-mounted display. Moreover, the display device 105 may be a plurality of displays, and for example, a display for a surgeon and a display for an assistant may be individually provided.

The microscope control input unit 106 receives the user's operation input into the microscope 102. For example, the user can perform an operation input such as moving the field of view of the microscope 102 in an arbitrary direction through the microscope control input unit 106. The microscope control input unit 106 is, for example, a foot switch.

The microscope control unit 107 controls the microscope 102 on the basis of an input signal fed from the microscope control input unit 106. The microscope control unit 107 is, for example, capable of adjusting the barrel position of the microscope 102 and the like in accordance with the input signal and moving the field of view of the microscope 102. Moreover, the magnification scale factor, the field of view, and the like of the microscope 102 can also be controlled by the microscope control unit 107.

The user input unit 108 receives an instruction input by the user. For example, the user input unit 108 is configured by an input device such as a mouse and a keyboard. In this embodiment, an instruction related to the generation and display of the magnified and reduced image described above and the like is input, for example. Additionally, information about details of a treatment to be performed by the user, surgical techniques of the treatment, and the like may be input. Furthermore, information that can be input by the user is not limited, and may be arbitrarily set.

The instrument 109 is an instrument such as a surgical instrument used for the treatment. The instrument 109 may include, for example, a laser probe, a vitreous cutter, and the like. In this embodiment, the image processing device 104 and the instrument 109 are connected to each other, and various types of information about the instrument 109 are input into the image processing device 104. For example, information such as the type of the instrument 109, an operation of the instrument, and a use condition of the instrument is input. Such information is included in treatment information.

It should be noted that although Fig. 1 shows the connection between the instrument 109 and the image processing device 104, the instrument 109 and the microscope control unit 107 may be connected to each other. The various types of information output from the instrument 109 may be used for controlling the microscope 102.

### [Functional Configuration of Image Processing Device]

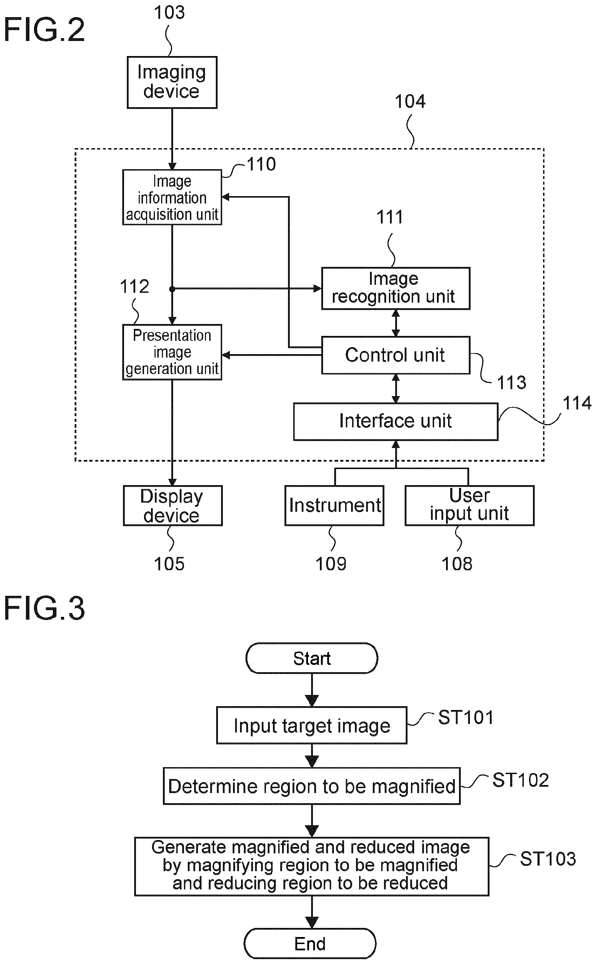

Fig. 2 is a block diagram showing a configuration example of the image processing device 104. The image processing device 104 includes hardware necessary for computer configurations such as a GPU, a CPU, a ROM, a RAM, and an HDD. An image processing method according to the present technology is performed by the CPU loading a program according to the present technology, which is recorded in advance in the ROM or the like, into the RAM and executing the program.

The image processing device 104 can be realized by any computer such as a personal computer (PC). Of course, hardware such as a GPU, an FPGA, and an ASIC may be used. Alternatively, the image processing device 104 may be realized by any computer system.

As shown in Fig. 2, in this embodiment, the image processing device 104 as a functional block realizes an image information acquisition unit 110, an image recognition unit 111, a presentation image generation unit 112, a control unit 113, and an interface unit 114 by the CPU executing a predetermined program. Of course, dedicated hardware such as an integrated circuit (IC) may be used for realizing each block.

The program is installed in the image processing device 104 via various recording media, for example. Alternatively, the program may be installed via the Internet, for example.

It should be noted that the type and the like of the recording medium on which the program is recorded is not limited, and any computer-readable recording medium may be used. For example, any recording medium for recording data in a non-transitory manner may be used.

The image information acquisition unit 110 generates, on the basis of the captured image from the imaging device 103, a target image that is an image including the eye to be treated. The target image can also be referred to as an image for which a magnified and reduced image is to be generated. For example, the captured image may be entirely used as the target image. Alternatively, a part of the captured image may be used as the target image. Moreover, for example, the region (outline) of the front lens in the captured image may be generated as the target image. That is, the image information acquisition unit 110 may acquire, as the target image, image information obtained by performing pre-processing on a captured image captured by the imaging device 103.

It should be noted that in the present disclosure, the image includes a still image and a moving image. Of course, the image also includes a plurality of frame images included in the moving image. Moreover, the acquisition of the image information includes acquisition of an image signal including the image information. Moreover, the data format of the image information and the like are not limited, and may be arbitrarily set.

The image recognition unit 111 performs image recognition processing on the target image acquired by the image information acquisition unit 110 under the control of the control unit 113. In this embodiment, the treatment information is detected by the image recognition processing of the image recognition unit 111.

The treatment information is information including at least one of information about the eye to be treated and information about the treatment to be performed on the eye as a target. For example, details of the treatment to be performed, surgical techniques to be performed, instruments (surgical instruments) to be used for the treatment, positions of parts of the surgical instruments, sites of the eye, a range to be illuminated (e.g., the centroid of an illumination region of the aiming beam or the like), a lesion, the range of the front lens 101, the outline of the target image, and the like are detected as the treatment information by image recognition. Of course, the present technology is not limited thereto, and at least one of those types of information or other information may be detected.

The specific method of detecting the treatment information by image recognition is not limited, and any technique may be used. For example, an arbitrary image recognition method such as edge detection and pattern matching may be used, and the algorithm is not particularly limited. Alternatively, any machine-learning algorithm using, for example, a deep neural network (DNN) may be used. For example, the use of artificial intelligence (AI) or the like that performs deep learning can improve the accuracy in detecting the treatment information.

It should be noted that the treatment information can also be generated on the basis of not only the target image, but also the captured image output from the imaging device 103.

Under the control of the control unit 113, the presentation image generation unit 112 generates an image (hereinafter, referred to as presentation image) displayed by the display device 105. In this embodiment, the magnified and reduced image is generated on the basis of the target image output from the image information acquisition unit 110 and is output as the presentation image.

The interface unit 114 receives various instructions input by the user input unit 108. The interface unit 114 also receives various types of information about the instrument 109 output from the instrument 109. The interface unit 114 receives the user's instructions and the information about the instrument 109 via, for example, a universal serial bus (USB) terminal, a high-definition multimedia interface (HDMI) (registered trademark) terminal, or the like.

Alternatively, the user's instructions and the information about the instrument 109 may be received by wireless LAN communication such as Wi-Fi or short-range wireless communication such as Bluetooth (registered trademark). That is, the communicable connection between the image processing device 104 and the user input unit 108 and the communicable connection between the image processing device 104 and the instrument 109 as shown in Fig. 1 may be realized with wires or may be realized wirelessly.

The control unit 113 controls each unit. For example, on the basis of an input instruction, the control unit 113 controls each unit by controlling the image recognition unit 111, the presentation image generation unit 112, and the interface unit 114 to generate a magnified and reduced image from an input target image.

Moreover, the control unit 113 is, for example, capable of performing determination of a surgical technique performed in a treatment on the basis of treatment information output from the image recognition unit 111 or the interface unit 114. It should be noted that the treatment information includes the recognition result of the image recognition unit 111 and the information about the instrument 109 as described above. The treatment information also includes a determination result in a determination made by the control unit 113 on the basis of such treatment information and a calculation result obtained in a predetermined calculation based on such treatment information.

In this embodiment, the image information acquisition unit 110 and the control unit 113 realize an image acquisition unit that acquires a target image including the eye to be treated. Moreover, the user input unit 108, the control unit 113, and the interface unit 114 can also be referred to as a reception unit configured to be capable of receiving the user's instruction.

Moreover, the presentation image generation unit 112 and the control unit 113 realizes an image processing unit that magnifies a first region in the target image and reduces a second region other than the first region in the target image such that the second region can be displayed in a region other than a magnified region that is the magnified first region in the target image. The operation of the image processing unit is an operation associated with the generation of the magnified and reduced image and will be described in detail below.

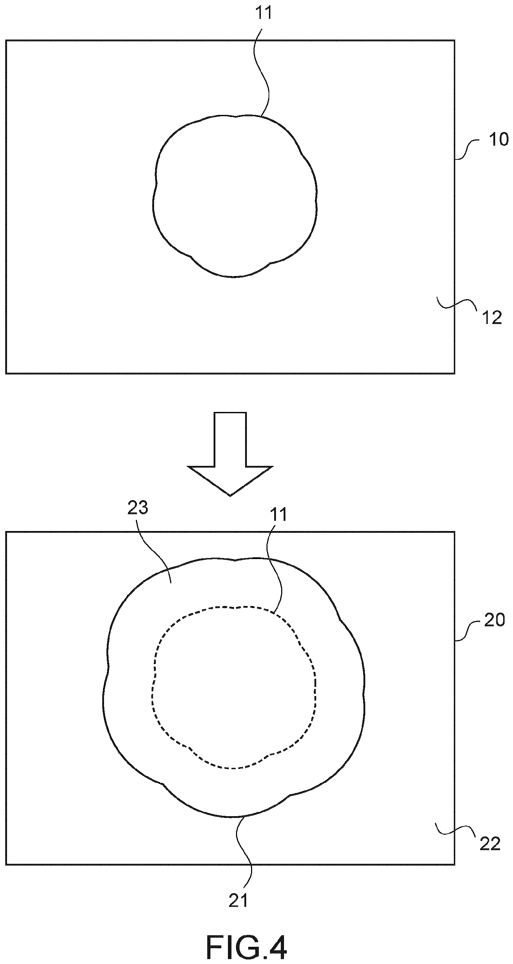

Fig. 3 is a flowchart showing a generation example of the magnified and reduced image. Fig. 4 is a schematic diagram for describing the generation of the magnified and reduced image. The image shown in the upper part of Fig. 4 is an example of the target image 10 and the image shown in the lower part is an example of the magnified and reduced image. It should be noted that in the target image 10 and the magnified and reduced image 20 shown in Fig. 4, the illustration of details of the image, such as the eye to be treated, are omitted.

First, the target image 10 is input (Step 101). As shown in Fig. 4, the region to be magnified 11 is determined with respect to the input target image 10 (Step 102). The region to be magnified 11 is typically a region on which the surgeon wishes to focus and is an important region in the treatment. Of course, the present technology is not limited to such a region.

The method of determining the region to be magnified is not limited, and any method may be employed. For example, the region to be magnified may be determined on the basis of an instruction input by the user. Alternatively, the region to be magnified may be automatically determined on the basis of the treatment information. For example, it is possible to determine the region to be magnified on the basis of details of the treatment, positions of parts of instruments, and the like.

A region other than the region to be magnified 11 in the target image 10 is a region to be reduced 12. That is, Step 102 can also be referred to as a step of determining the region to be magnified 11 and the region to be reduced 12. It should be noted that in this embodiment, the region to be magnified corresponds to the first region and the region to be reduced corresponds to the second region.

The magnified and reduced image 20 is generated by magnifying the region to be magnified 11 and reducing the region to be reduced 12 (Step 103). Specifically, the region to be magnified 11 in the target image 10 is magnified as shown in Fig. 4. Then, the region to be reduced 12 in the target image 10 is reduced to be capable of being displayed in a region 22 other than a magnified region 21 that is the magnified region to be magnified in the target image 10 (i.e., the magnified region to be magnified after magnification).

The magnified and reduced image 20 is generated by magnifying the region to be magnified 11 and reducing the region to be reduced 12 other than the region to be magnified 11 in that manner. This makes it possible to achieve both a magnifying view of the region to be magnified 11 and a check of the entire image (operative field) without losing the details (information) of the entire original image. As a result, it is possible to sufficiently grasp the target image 10 including the eye to be treated.

It should be noted that in the present disclosure, magnifying an image means displaying (details of) the image in a region larger than an original display region. Reducing an image means displaying (details of) the image in a region smaller than the original display region.

The image magnifying method for displaying the image in the region larger than the original display region and the image reducing method for displaying the image in the region smaller than the original display region are not limited. For example, the entire region to be magnified 11 may be magnified by a predetermined scale factor. Alternatively, different scale factors may be respectively assigned to a plurality of regions in the region to be magnified 11, and the magnification processing may be performed in accordance with the assigned scale factors. Alternatively, the region to be magnified may be entirely displayed in the region larger than the original display region by magnifying only a partial region of the region to be magnified 11. Even in a case where different magnification scale factors (including same scale factor) are respectively set, it is possible to sufficiently prevent the image from being damaged by performing arbitrary image processing such as interpolation processing based on pixel information.

Also regarding the image reducing method, the entire region to be reduced 12 may be reduced at a predetermined scale factor, for example. Alternatively, different scale factors may be respectively assigned to a plurality of regions in the region to be reduced 12, and the reduction processing may be performed in accordance with the assigned scale factors. Alternatively, the region to be reduced may be entirely displayed in the region smaller than the original display region by reducing only a part of the region to be reduced 12. Even in a case where the different reduction scale factors (including same scale factor) are set respectively, it is possible to sufficiently prevent the image from being by performing arbitrary image processing such as interpolation processing based on pixel information.

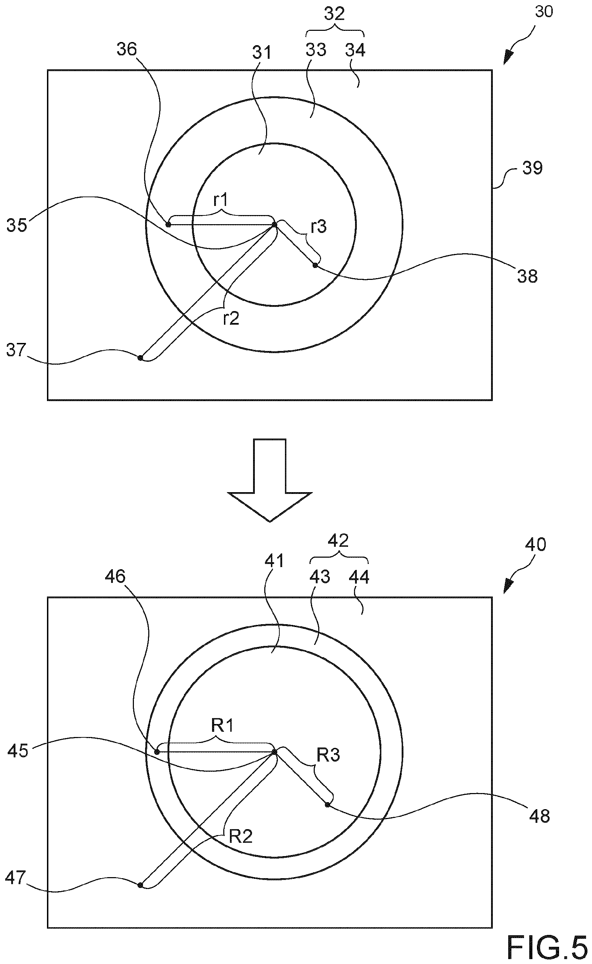

Fig. 5 is a diagram for describing an example of a magnification method for a region to be magnified 31 and a reduction method for a region to be reduced 32. For example, as shown in Fig. 5, a peripheral region 33 adjacent to the periphery of the region to be magnified 31 and a separate region 34 other than the peripheral region 33 are set to the region to be reduced 32. Then, the entire region to be reduced is reduced by reducing the peripheral region 33. Therefore, regarding the separate region 34, the same image (image at the same scale factor) as the target image 30 is displayed as it is.

Moreover, a predetermined position in the region to be magnified 31 is set as a reference position 35 as shown in Fig. 5. For example, the reference position 35 is a position serving as a reference for magnification processing of the region to be magnified 31. For example, the region to be magnified 31 may be magnified such that the distance between the reference position 35 and each position of the region to be magnified 31 is increased.

Moreover, for example, the same reference position 35 may be a position used as a reference for the reduction processing of the region to be reduced 32. For example, the region to be reduced 32 may be reduced such that the distance between the reference position 35 and each position of the separate region 34 is maintained. In this case, the region to be reduced 32 is reduced such that each position in the peripheral region 33 is included between the edge of the magnified region 31 and the boundary between the peripheral region 33 and the separate region 34.

It should be noted that an outermost portion 39 of the target image 30 is included in the separate region 34. Thus, the distance between the reference position 35 and each position of the outermost portion 39 of the target image 30 is maintained. For example, it is assumed that the peripheral region 33 and the separate region 34 are not set and the entire region to be reduced 32 is reduced. Even in this case, the region to be reduced 32 is reduced such that the distance between the reference position 35 and each position of the outermost portion 39 of the target image 30 is maintained. This makes it possible to prevent image missing at the edge of the target image 30.

By performing scaling processing on each of the above-mentioned positions, the magnified and reduced image 40 including a reference position 45 having coordinates common to those of the reference position 35, a magnified region 41 in which the region to be magnified 31 has been magnified, a reduced peripheral region 43 in which the peripheral region 33 has been reduced, and a separate region 44 in which the distance between the reference position 45 and each position is maintained is generated. It should be noted that the reduced peripheral region 43 and the separate region 44 can also be referred to as a reduced region 42.

It should be noted that in this embodiment, the peripheral region 33 corresponds to a peripheral region adjacent to the periphery of the first region. Moreover, the separate region 34 corresponds to a region other than the peripheral region.

Here, the scale factor of each position of the original target image 30 is defined as follows.

### (Distance from the reference position 45 in the magnified and reduced image 40)/(distance from the reference position 35 in the original target image 30)

For example, the scale factor of a position 36 of the target image 30, which corresponds to a position 46 of the magnified and reduced image 40, is R1/r1. Similarly, the scale factor of a position 37 of the target image 30, which corresponds to a position 47 of the magnified and reduced image 40, is R2/r2. Also, the scale factor of a position 38 of the target image 30, which corresponds to a position 48 of the magnified and reduced image 40, is R2/r2.

Each position of the region to be magnified 31 is set such that the scale factor is larger than 1. For each position of the separate region 34, the scale factor is set to be 1. For the peripheral region 33, the scale factor of each position is set as appropriate to shift from the scale factor (greater than 1) of each position of the edge of the magnified region 41 to the scale factor (equal to 1) of each position of the boundary between the peripheral region 33 and the separate region 34.

It should be noted that in the present disclosure, it is assumed that the boundary between the two regions is included in each of the two regions. Therefore, it is assumed that the boundary between the peripheral region 33 and the separate region 34 is included in both the peripheral region 33 and the separate region 34.

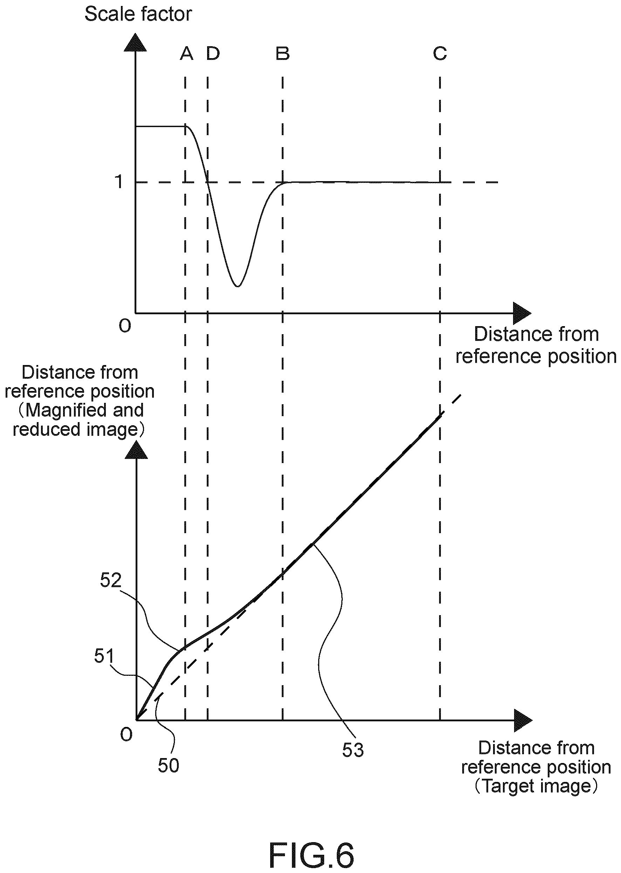

Fig. 6 show graphs for describing an example of setting the scale factor of each position. As shown in Fig. 6, the magnified and reduced image 40 can be generated by setting the scale factor of each position of the region to be magnified 31 and the scale factor of each position of the region to be reduced 32 (the peripheral region 33 and the separate region 34).

The vertical axis of the upper graph of Fig. 6 indicates the scale factor of the magnification or reduction performed on the target image 30. Also, the horizontal axis indicates the distance from the reference position 35. That is, the upper graph of Fig. 6 is a graph showing a change in scale factor of the region to be magnified 31 and the region to be reduced 32 on the basis of the distance from the reference position 35.

As shown in the upper graph of Fig. 6, the range from the reference position 35 to a distance A is magnified at a high scale factor. Hereinafter, the range from the reference position 35 to the distance A will be referred to as a high-scale factor portion. Moreover, the scale factor of the high-scale factor portion is arbitrarily determined. For example, in a case where the user wishes to more specifically grasp one portion, the magnification may be performed at a higher scale factor.

Moreover, regarding the range between the distance A to a distance B, the processing switches from the magnification to the reduction at a distance D. That is, the region from the reference position 35 to the distance D is the region to be magnified 31. The point of the distance D from the reference position 35 can also be referred to as the edge of the region to be magnified 31.

Between the distance D to the distance B, the region around the region to be magnified 31 is reduced. That is, the region between the distance D to the distance B is the peripheral region 33. Moreover, the scale factor is 1 between the distance B to the distance C. The distance C represents an image edge of the target image 30 and the magnified and reduced image 40. That is, the region between the distance B to the distance C is the separate region 34.

It should be noted that in this embodiment, the image edge of the target image 30 corresponds to each position of the outermost portion of the target image 30. That is, the presentation image generation unit 112 reduces the region to be reduced 32 such that the distance between each position of the outermost portion of the reference position 35 and the target image 30 is maintained.

The lower graph of Fig. 6 is a graph showing the distance from the reference position 35 in the target image 30 and the distance from the reference position 35 in the magnified and reduced image 40. The horizontal axis indicates the distance from the reference position 35 of the target image 30. Moreover, the vertical axis indicates the distance from the reference position 45 of the magnified and reduced image 40.

As shown in the lower graph of Fig. 6, the dotted line 50 is a straight line with a gradient of 1. That is, the dotted line 50 indicates a position at which the distance from the reference position 35 in the target image 30 and the distance from the reference position 35 in the magnified and reduced image 40 are equal, and shows a state in which the target image 30 and the magnified and reduced image 40 are at the same scale factor. Moreover, in a case where the dotted line 50 and the solid line overlap each other, the positional relationship between the corresponding positions in the target image 30 and the magnified and reduced image 40 is the same.

The solid line 51 from the reference position 35 to the distance D is a curve with a gradient greater than 1. That is, within the magnified region 31 from the reference position 35 to the distance D, the distance from the reference position 35 in the magnified and reduced image 40 is longer than the distance from the reference position 35 of the target image 30 because of the magnification. Moreover, the solid line 51 has a constant gradient from the reference position 35 to the distance A because of the constant scale factor.

The solid line 52 from distance D to distance B is a curve with a gradient less than 1. That is, within the reduced region 42 from the distance D to the distance B, the solid line 52 approaches the position at which the distance from the reference position 35 in the target image 30 and the distance from the reference position 35 in the magnified and reduced image 40 are equal because of the reduction.

Moreover, the solid line 53 coincides with the dotted line 50 at the distance B. That is, the positional relationship between each position of the separate region 34 in the target image 30 and each position of the separate region 34 in the magnified and reduced image 40 is the same.

In this embodiment, the control unit 113 determines the length of each of the regions from the reference position to the distance A, the distance B, the distance C, and the distance D. For example, the control unit 113 determines the length of each region on the basis of the type of a surgical technique input from the interface unit 114.

In order to prevent image missing within the range of the magnified and reduced image 40, it is necessary that the relationship between the distance from the reference position 35 in the target image 30 and the distance from the reference position 35 in the magnified and reduced image 40 as in the lower graph of Fig. 6 is continuous and the portion at which the relationship between the distance from the reference position 35 in the target image 30 and the distance from the reference position 35 in the magnified and reduced image 40 becomes the same, i.e., the point at which the dotted line 50 and the solid line 53 coincide with each other, is located within the image range (from the reference position 35 to the distance C).

Moreover, image missing occurs in a case where the point at which the dotted line 50 and the solid line 53 coincide with each other, is not located within the image range. For example, in a case where the solid line exceeds the dotted line 50 by the distance C, the image edge of the target image 30 departs from the image range in the magnified and reduced image 40 and image missing occurs.

As shown in the upper graph of Fig. 6, the positional relationship from the reference position 35 as shown in the lower graph of Fig. 6 is first determined as the way for determining a scale factor at each position of the target image 30. Then, the scale factor at each position in the upper graph of Fig. 6 is determined by differentiating positions of pixels of the magnified and reduced image 40 in the lower graph of Fig. 6 with respect to positions of pixels of the target image 30, which is easy in terms of design.

It should be noted that the image edge may be arbitrarily set. For example, the position of the outline of the front lens 101 may be set as the image edge such that the magnification and reduction processing is completed within the range of the front lens 101, which is an important operative field with a high degree of interest for the user.

It should be noted that the shape and area of the region to be magnified 31 are not limited. The user may determine the region to be magnified 31 to have arbitrary shape and area.

It should be noted that the scale factor is not limited. For example, the scale factor may be determined in accordance with the direction from the reference position. For example, the scale factor in a direction opposite to a direction in which the surgical instrument's shaft is located may be set to be high. That is, the scale factor may be determined by estimating the degree of interest (degree of difficulty in viewing) for the user on the basis of the shadow of the surgical instrument and the like.

It should be noted that a predetermined pixel in the target image 30 can be set as the reference position. Then, as the position of each region, each pixel included in the region can be set. The magnified and reduced image can be generated on the basis of a distance between a pixel (hereinafter, referred to as a reference pixel) that is the reference position and each pixel of each region.

It should be noted that there may be a case where there is no pixel at a position in the magnified and reduced image 40, at which the distance from the reference pixel has been increased or reduced. In this case, the magnified and reduced image 40 can be generated without image missing by performing interpolation processing of the pixel information with pixels surrounding that position, for example.

Moreover, regarding the setting of the reference position 35 in the region to be magnified 31, the reference position may be set at a predetermined position such as the center of the region to be magnified 31 after the region to be magnified 31 is set. Alternatively, after the reference position is set, the region to be magnified 31 may be set using the reference position 35 as the reference. In this case, the determination of the region to be magnified 31 and the setting of the reference position 35 can be considered as equivalent processing. Of course, the method of generating the magnified and reduced image 40 is not limited, and any other method may be employed.

There may also be a case where magnified and reduced images are continuously generated for a plurality of frame images (a plurality of target images) constituting a moving image. In this case, the region to be magnified (reference position) may be individually set for each target image. That is, there may also be a case where the region to be magnified (reference position) is set to dynamically change for each target image.

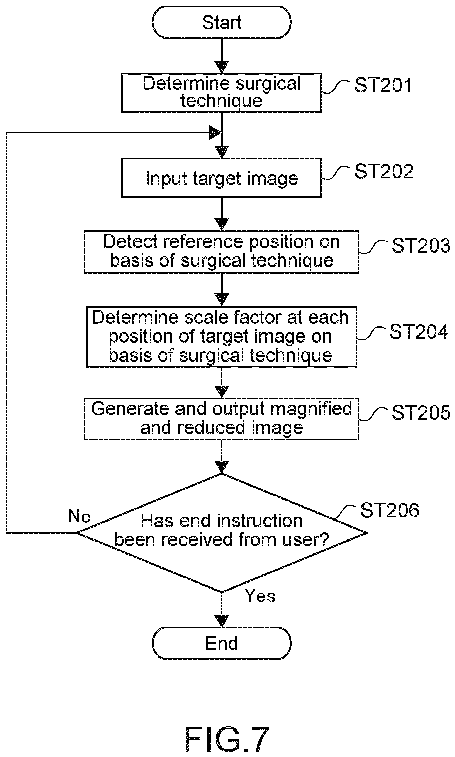

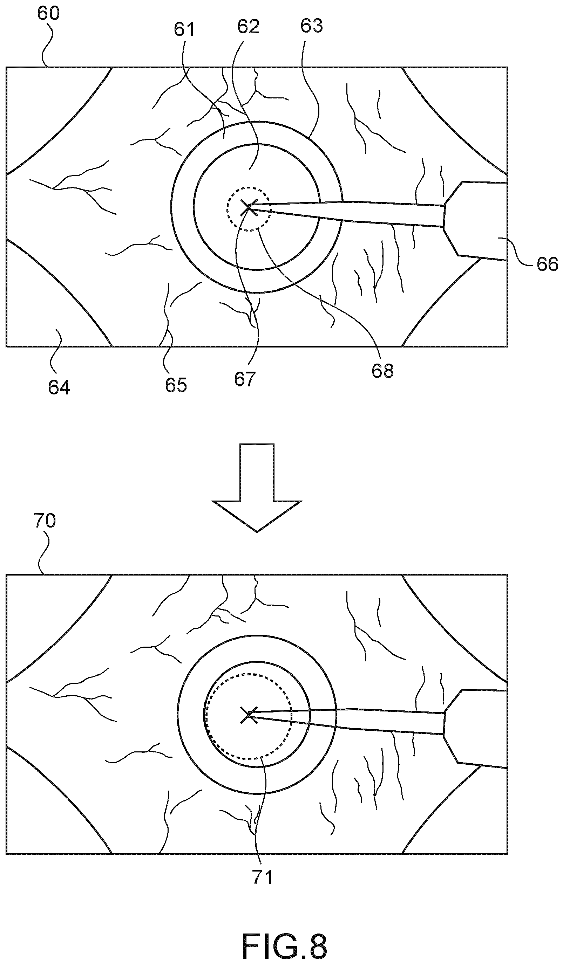

Fig. 7 is a flowchart showing a specific example of processing of generating a magnified and reduced image 70. Fig. 8 is a schematic diagram showing a change in scale factor of a target image 60 obtained by imaging an eye to be treated. In the above description, the image obtained through the front lens is set as the processing target because the assumed case is the intraocular imaging. Otherwise, in a case where an anterior ocular segment is imaged as in the case shown in Fig. 8, it is favorable to prevent the front lens from being located within the imaging range by setting the position of the front lens outside the optical path or removing the front lens from the system, for example.

During the treatment, the target of interest of the surgeon differs depending on a surgical technique and the degree of interest also differs depending on the position of the operative field. Therefore, in order to improve the convenience of the surgeon, it is desirable that estimation of the target of interest of the surgeon and determination of the scale factor of the image at each position as a result be performed on the basis of the surgical technique. Hereinafter, a specific example of processing including such determination of the scale factor will be described.

The user inputs into the interface unit 114 information about a surgical technique to be performed on the eye as a target. The control unit 113 determines the surgical technique to be performed on the basis of the input information (Step 201). For example, in a case of performing a surgical technique for removing the vitreous body of a patient, the user inputs into the interface unit 114 information indicating that the vitrectomy is to be performed.

It should be noted that a determination method of the surgical technique is not limited. For example, the surgical technique may be determined by receiving a signal indicating that a particular instrument for performing the surgical technique is connected to the surgical microscope system 100, or the like. Moreover, the surgical technique may be determined on the basis of information about a device operation mode (e.g., a laser coagulation mode or a vitrectomy mode) from a particular instrument, for example, from a vitreous surgical device. For example, the surgical technique is displayed in a menu on the display device 105 and the user can designate the surgical technique via the input device.

It should be noted that in this embodiment, the information about the surgical technique corresponds to the information about the treatment to be performed on the eye as a target. It should be noted that in this embodiment, the surgical technique corresponds to the details of the treatment.

The target image 60 acquired by the image information acquisition unit 110 is input into the image recognition unit 111 (Step 202).

As shown in Fig. 8, the target image 60 obtained by imaging an iris 61, a pupil 62, a limbus 63, an eyelid 64, a blood vessel 65, and a surgical instrument 66 of the eye is displayed by the display device 105. That is, the target image 30 corresponds to the operative field of the person who performs the treatment.

In this embodiment, the control unit 113 detects a reference position 67 at a tip position of the surgical instrument 66 on the basis of the surgical technique input from the interface unit 114 (Step 203).

It should be noted that in this embodiment, the iris 61, the pupil 62, the limbus 63, the eyelid 64, and the blood vessel 65 of the eye correspond to the information about the eye to be treated. Moreover, in this embodiment, the surgical instrument 66 corresponds to an instrument used for the treatment.

Moreover, the image recognition unit 111 and the interface unit 114 can also be referred to as an information acquisition unit that acquires treatment information including at least one of the information about the eye to be treated or information about the treatment to be performed on the eye as a target.

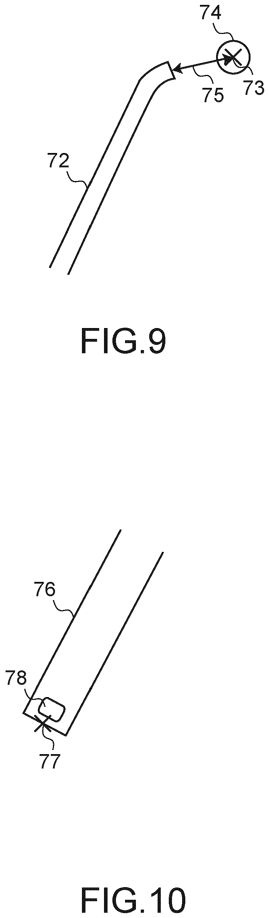

Figs. 9 to 11 are used for showing an example of detection of a reference position based on each surgical technique.

Fig. 9 is a schematic diagram showing an example of detection of the reference position at the time of laser coagulation. The laser coagulation refers to a surgical technique of coagulating a retina through laser irradiation for a retinal disease. The laser coagulation is used for burning new blood vessels and for fixing the periphery of a retinal tear to prevent retinal detachment from occurring.

As shown in Fig. 9, a reference position 73 is detected at the position of an aiming beam radiated from a laser probe 72. The aiming beam is radiated to a part of the retina, which is wished to be coagulated. That is, the reference position 73 is detected within a range illuminated with the aiming beam, which is the target of interest of the user. In this embodiment, the reference position 73 is detected at the centroid of a range to be illuminated 74.

It should be noted that the method of detecting the reference position 73 at the time of laser coagulation is not limited. For example, the reference position 73 may be detected at a position of a constant distance 75 from the tip of the laser probe 72 in the tip orientation. Moreover, for example, the reference position may be set at the tip of the laser probe 72.

It should be noted that the setting of the distance 75 used for detecting the reference position is not limited. For example, the distance 75 used for detecting the reference position 73 may be a predetermined fixed value in a range of 0 to 5 mm or may be dynamically changed in a range of 0 to 5 mm during the treatment.

Moreover, for example, a thickness of the laser probe 72, an optical zoom magnification of the microscope, and a distance in the image may be references for setting the range of the distance 75. A distance at which the tip of the surgical instrument such as the laser probe 72 and the depth of the retina are estimated by stereoscopic vision using stereo images of the microscope, which correspond to both eyes, may be used as a reference.

Here, the distance used for detecting the reference position may be a distance in a three-dimensional space or may be a distance as projected orthographically in a direction of the image plane.

Fig. 10 is a schematic diagram showing an example of detection of a reference position at the time of removing the vitreous body. The vitrectomy refers to a surgical technique of removing the vitreous body through a vitreous cutter for removing the vitreous body in the eyeball. For example, the vitrectomy is performed for removing and aspirating vitreous bleeding, haze, etc. to make the vitreous body clear.

As shown in Fig. 10, a reference position 77 is detected at the tip of a vitreous cutter 76. It should be noted that the method of detecting the reference position at the time of removing the vitreous body is not limited. For example, the reference position 77 may be detected at a position at a constant distance from an opening 78 of a vitreous cutter 76 or the tip of the vitreous cutter 76.

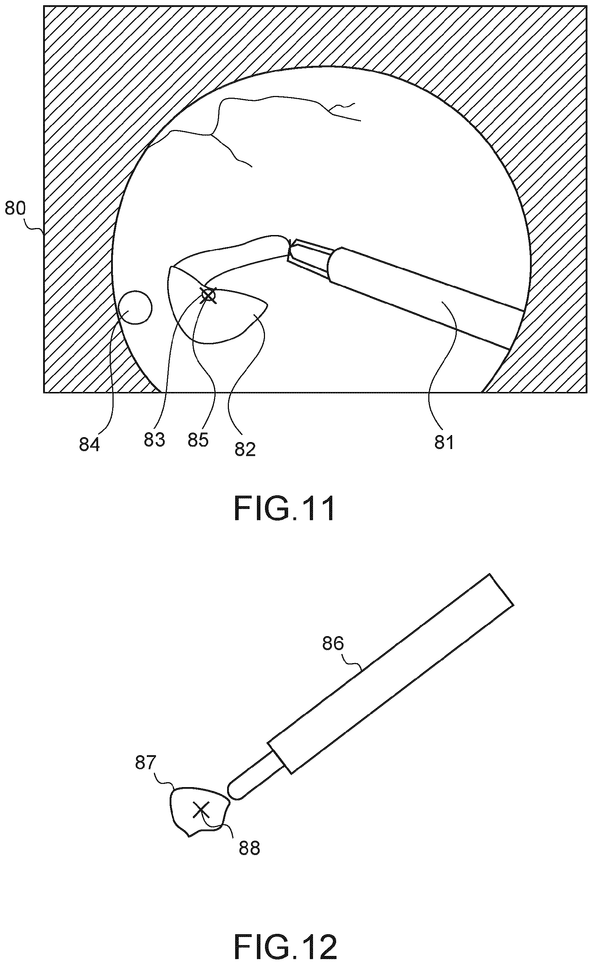

Fig. 11 is a schematic diagram showing an example of detection of a reference position at the time of inner boundary membrane peeling. Internal limiting membrane peeling refers to a surgical technique of peeling an inner limiting membrane. For example, the inner limiting membrane peeling is performed in a macular hole in which a hole is formed in the retina of the fovea of the fundus or an epiretinal membrane of the macula in which the macula is blocked by a membrane developing in front of the retina in the posterior part of the eyeball.

As shown in Fig. 11, the image information acquisition unit 110 acquires a target image 80 obtained by imaging ILM forceps 81, a macula 82, a fovea 83, and an optic disc 84. The control unit 113 detects a reference position 85 at the position of the fovea 83 of the macula 82 on the retina.

It should be noted that the method of detecting the reference position 85 during the inner limiting membrane peeling is not limited. For example, the reference position 85 may be detected at a midpoint of the tips of the ILM forceps 81 or at an internally dividing point between the fovea 83 and the tips of the ILM forceps 81. Moreover, a reference position similar to that at the time of peeling the inner limiting membrane may be used as a reference position at the time of peeling the epiretinal membrane of the macula.

Fig. 12 is a schematic diagram showing an example of detection of the reference position at the time of drainage of subretinal fluid. The drainage of subretinal fluid refers to a surgical technique of removing fluid accumulated under the retina (subretinal fluid). For example, the drainage of subretinal fluid is performed in a case where a treatment for retinal detachment or the like is performed.

As shown in Fig. 12, the image information acquisition unit 110 acquires a target image obtained by imaging a backflash needle 86 and a retinal tear hole 87. The control unit 113 detects a reference position 88 at the center position in the range of the retinal tear hole 87 located in the vicinity of the backflash needle 86.

It should be noted that the method of detecting the reference position 88 at the time of drainage of subretinal fluid is not limited. For example, the reference position 88 may be detected at the tip of the backflash needle 86. Also, for example, the reference position 88 may be detected at an internally dividing point between the centroid of the region of the retinal tear hole 87 and the tip of the backflash needle 86.

It should be noted that in this embodiment, the retinal tear hole 87 corresponds to the lesion. Moreover, disease states of the eye, such as retinal detachment and new blood vessels, which are variously treated, correspond to the lesions.

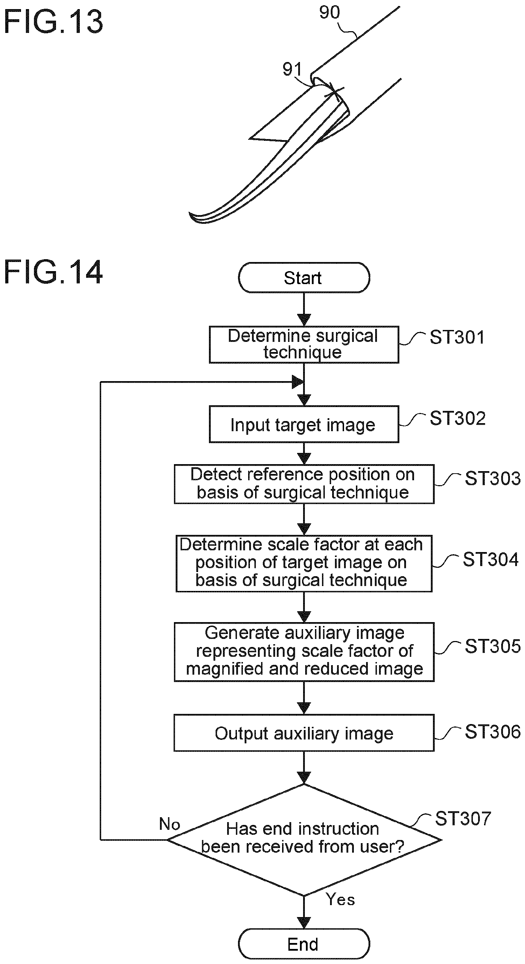

Fig. 13 is a schematic diagram showing an example of detection of a reference position at the time of removing a proliferative membrane. The proliferative membrane removal refers to a surgical technique of removing the proliferative membrane adhering to the retina. For example, the proliferative membrane removal is performed when removing a proliferative membrane in proliferative diabetic retinopathy.

As shown in Fig. 13, a reference position 91 is detected in a pipe tip portion of retinal scissors 90. It should be noted that the method of detecting the reference position 91 at the time of removing the proliferative membrane is not limited. For example, the reference position 91 may be detected at the tips of the retinal scissors 90 or the reference position 91 may be detected at the internally dividing point between the pipe tip portion of the retinal scissors 90 and the tips of the retinal scissors 90.

It should be noted that the method of detecting the reference position 35 is not limited. For example, the reference position 35 may be determined on the basis of an instruction input by the user. Moreover, for example, on the assumption that the user is focusing on the tip of the surgical instrument, the tip of the surgical instrument may constantly be set as the reference position 35. Of course, it is not limited to the above-mentioned surgical instrument, and the method of detecting the reference position may be set for each of various surgical instruments such as an intraocular illumination fiber.

The control unit 113 determines a scale factor at each position of the target image on the basis of the detected reference position (Step 204). In this embodiment, the control unit 113 sets the region to be magnified 31 in a circular shape centered at the reference position 35. That is, the presentation image generation unit 112 magnifies the region to be magnified 31, using the reference position 35 as the reference.

The presentation image generation unit 112 generates the magnified and reduced image 70 with respect to the target image 60 in accordance with the control of the control unit 113. That is, the presentation image generation unit 112 magnifies a region to be magnified 68 and reduces a region other than the region to be magnified 68 to be capable of being displayed in a region other than a magnified region 71 that is the magnified region to be magnified 68 in the target image 60.

Moreover, the magnification and reduction processing in a region farther from the reference position 67 than the region to be reduced is regulated. That is, the positional relationship between the target image 60 and the magnified and reduced image 70 becomes the same in the region farther from the reference position 67 than the region to be reduced. For example, a region outside the limbus 63 from the reference position 67, which includes the eyelid 64, the blood vessel 65, and the like, is displayed such that the positional relationship between the target image 60 and the magnified and reduced image 70 becomes the same.

The presentation image generation unit 112 generates the magnified and reduced image 70 on the basis of the determined scale factor at each position and displays the generated magnified and reduced image 70 on the display device 105 (Step 205).

In a case where an end instruction to terminate magnification and reduction is input into the interface unit 114, the control unit 113 terminates the generation of the magnified and reduced image 70 (Step 206). In a case where the user needs a new magnified and reduced image 70 or the like, an instruction to regenerate the magnified and reduced image 70 is input into the interface unit 114, and the processing accordingly returns to Step 202 (No in Step 206).

It should be noted that the order of processes in which the magnified and reduced image 70 is generated is not limited. For example, the determination of the surgical technique of Step 201 may be performed after the input of the target image of Step 202. In this case, the determination method for the surgical technique may be determined on the basis of information obtained from the target image.

For example, as shown in Fig. 9, in a case where the laser probe 72 is displayed in the input target image and the laser radiated from the laser probe 72 is radiating a lesion such as a retinal tear, the surgical technique is determined to be laser coagulation.

Moreover, for example, as shown in Fig. 11, it is determined that the surgical technique is being performed on the retinal vitreous body because the periphery of the target image 80 is dark and it is determined that the surgical technique is the inner limiting membrane peeling because the ILM macula 81 is used and the tip of the ILM macula 81 is close to the macula 82.

That is, the determination of the surgical technique may be performed on the basis of the type of the surgical instrument, the position and movement of the tip of the surgical instrument, the lesion of the eye, or the like in the target image.

As described above, in the image processing device 104 according to this embodiment, the target image 10 including the eye to be treated is acquired. The region to be magnified 11 in the target image 10 is magnified, and the region to be reduced 12 other than the region to be magnified 11 in the target image 10 is reduced to be capable of being displayed in the region 22 other than the magnified region 21 which is the magnified region to be magnified 11 in the target image 10. Accordingly, it is possible to sufficiently grasp the target image 10 including the eye to be treated.

In ophthalmologic surgery, there is a demand for magnifying the operative field in order to perform a minute surgical techniques, while there is a contradictory demand for grasping the situation of the entire operative field including an area other than the operative position. The latter is remarkable in a case of performing surgery while observing a wide region of the retina by using a wide-angle observation system.

As an example of ophthalmologic surgery, in a case of performing a surgical technique by using a surgical instrument such as forceps and a vitreous cutter, the vitreous body may get caught in the surgical instrument, for example, and the retina may be accordingly pulled in a site different from the site where the surgical technique is being performed. As a result, retinal tears, retinal detachment, and the like may occur.

As such, another eye disorder such as a complication may arise at a position different from the position of the treatment target in a case of performing an intraocular treatment, and therefore it is desirable to grasp the state of the entire eye including the position of the treatment target.

Therefore, in the present technology, the operative field image is displayed without image missing by estimating the portion of interest of the user and reducing the peripheral portion thereof while magnifying the same portion. Thus, both the magnification vision and the grasping of the entire operative field are realized.

Accordingly, magnifying the target of interest of the user while grasping the entire state of the target image (operative field) at the time of performing various surgical techniques in eye surgery enables the target to be observed in detail.

### <Second Embodiment>

When the magnified and reduced image is generated and displayed as in the first embodiment, there is a possibility that the user may be confused because the user cannot recognize which part of the displayed image is magnified or reduced. A surgical microscope system according to a second embodiment of the present technology will be described below as an example of avoiding such confusion and supporting recognition of a displayed operative field. In the following description, descriptions of a configuration and an operation similar to those of the surgical microscope system 100 described in the embodiment above will be omitted or simplified.

In the first embodiment, the presentation image generation unit 112 generates the magnified and reduced image obtained by changing the scale factor of the target image. In the second embodiment, the presentation image generation unit 112 generates the magnified and reduced image and an auxiliary image representing the scale factor of the magnified and reduced image.

Fig. 14 is a flowchart showing the processing of the presentation image with respect to the scale factor at each position of the magnified and reduced image of the second embodiment according to the present technology. The processing from Step 301 to Step 304 is similar to the processing from Step 201 to Step 204 described in the first embodiment, and therefore the description thereof will be omitted.

As shown in Fig. 14, the presentation image generation unit 112 generates an auxiliary image representing a scale factor of a magnified and reduced image (Step 305). The auxiliary image generated by the presentation image generation unit 112 is fed to the display device 105 to thereby be displayed on a display or the like (Step 306).

Moreover, the presentation image generation unit 112 can also be referred to as be a generation unit capable of generating an auxiliary image representing a state of magnification with respect to the first region and a state of reduction with respect to the second region.

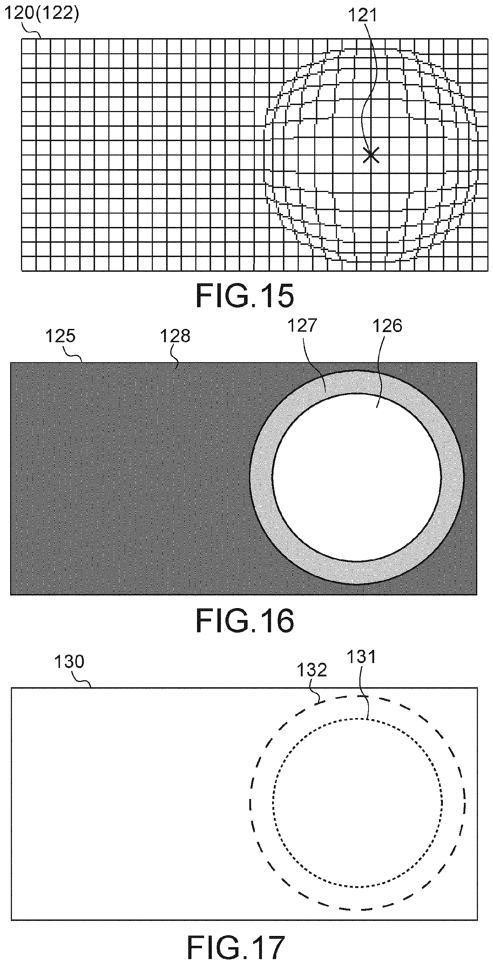

Figs. 15 to 18 are schematic diagrams showing an example of an auxiliary image representing the scale factor at each position of the magnified and reduced image.

As shown in Fig. 15, it is assumed that a reference position 121 is detected in a case where a captured target image 120 is an image in which grid lines are drawn. The presentation image generation unit 112 generates an auxiliary image 122 indicating what magnified and reduced image is generated in a case where the target image 120 is processed at the scale factor determined on the basis of the reference position 121 into a magnified and reduced image.

The display device 105 displays the auxiliary image 122 in a state of being overlaid on the magnified and reduced image. It should be noted that the image itself in a state in which the auxiliary image 122 is overlaid on the magnified and reduced image may be used as the auxiliary image. Here, the overlay includes superimposing and displaying two images. The superimposition of two images can also be referred to as be superimposed.

As shown in Fig. 16, an auxiliary image 125 in which the pixel values of the magnified and reduced image are processed is generated on the basis of the scale factor at each position of the magnified and reduced image. For example, a magnified region 126 and a reduced region 127 and an same-scale factor region 128 are processed and displayed to be different in luminance, saturation, or hue. That is, a magnified region (a region at a scale factor larger than the same scale factor) and a reduced region (a region at a scale factor smaller than the same scale factor) are processed and displayed to be different in the luminance, saturation, or hue in the same-scale factor region 128. For example, a method of adding a fixed value to a luminance value in each region, multiplying a luminance value in each region by a fixed value, or the like can be considered as a processing method. Moreover, for example, a method of replacing a value of a color difference, a hue value, a saturation value, or the like for each region with a fixed value, a method of adding a fixed value for each region to the value or multiplying the fixed value for each region by the value, or the like is conceivable as another method of processing.

The presentation image generation unit 112 provides the generated auxiliary image 125 to the display device 105 instead of the magnified and reduced image. It should be noted that the number of regions of the auxiliary image 125 is not limited. For example, the region of the auxiliary image may be color-coded in four regions: the high-scale factor portion, the magnified region 126, the reduced region 127, and the same-scale factor region 128.

It should be noted that a method of displaying different regions is not limited. For example, other color space values such as YIQ, HVS, and LCH may be used also for a color space other than a color space defined by YCbCr values as the space defining the brightness, saturation, and hue, and also a color space as a base may be Lab, Lch, XYZ, or the like other than RGB.

Moreover, for example, processing of the luminance value and the colors may be performed on the basis of the scale factor of each pixel of the magnified and reduced image. In the processing related to the luminance value, the value of the scale factor or the converted value may be added or multiplied with respect to the luminance value of each pixel, for example. In the processing related to the colors, for each pixel, a scale factor value at the position or its converted value may be added or multiplied with respect to the color difference, the hue value, or the saturation value, for example.

As shown in Fig. 17, an auxiliary image 130 displaying a frame line indicating the scale factor in each region of the magnified and reduced image is generated. For example, a frame line 131 indicating the edge of the magnified region and a frame line 132 indicating the edge of the reduced region are displayed in the auxiliary image 130.

The display device 105 displays the auxiliary image 130 in a state of being overlaid on the magnified and reduced image. It should be noted that the number of frame lines and the like are not limited, and a frame line indicating a high-scale factor portion may be displayed.

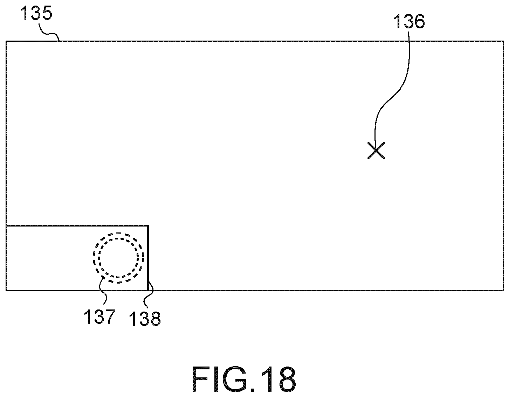

As shown in Fig. 18, at least one of the auxiliary images generated in Figs. 15 to 17 is displayed on a part of a magnified and reduced image 135 by using picture-in-picture (PiP). For example, an auxiliary image 137 is reduced and displayed outside the range of the magnified region and the reduced region with reference to a reference position 136. That is, the auxiliary image 137 is displayed outside the region in the magnified and reduced image 135, on which the user is focusing.

In a case where an end instruction to terminate the presentation of the auxiliary image is input into the interface unit 114, the control unit 113 terminates the output of the auxiliary image (Yes in Step 307). In a case where the user needs a new auxiliary image or the like, an instruction to regenerate the auxiliary image is input into the interface unit 114, to thereby the processing accordingly returns to Step 202 (No in Step 307). That is, the user can arbitrarily switch the display of the auxiliary image.

It should be noted that the place where the auxiliary image 137 is displayed and the reduction scale factor is not limited. For example, the size and position of a frame 138, in which the auxiliary image 137 is displayed, and the reduction scale factor of the auxiliary image 137 may be automatically determined or arbitrarily determined by the user.

Moreover, a magnified and reduced image in which a magnified and reduced image or an auxiliary image is overlaid may be displayed, and a target image may be displayed in the frame 138. That is, the image (inside the frame 138) before the region to be magnified is magnified and the region to be reduced is reduced and the image (other than the inside of the frame 138 of the presentation image) after the region to be magnified is magnified and the region to be reduced is reduced can be compared to each other.

Accordingly, the use of the auxiliary image indicating which portion in the magnified and reduced image is magnified and which portion in the magnified and reduced image is magnified is reduced can prevent the user from being confused when the user recognizes the operative field.

### <Other Embodiments>

The present technology is not limited to the above-mentioned embodiments, and various other embodiments can be realized.

In the first and second embodiments described above, the reference position is set and the region to be magnified is magnified, using the reference position as the reference. The present technology is not limited thereto, the region to be magnified may be arbitrarily determined by the user. That is, in a case where the user arbitrarily determines the region to be magnified, the detection of the reference position does not need to be performed.

In the first embodiment, the setting of the high-scale factor portion is arbitrarily set. The present technology is not limited thereto, the reference position may be set on the basis of the detected reference position. For example, in the case of the inner limiting membrane peeling shown in Fig. 11, the range of the high-scale factor portion may be set on the basis of the distance between a first reference position detected in the fovea 83 (the same position as the reference position 85) and a second reference position detected at the tip of the ILM macula 81. Of course, the range of the high-scale factor portion may be set to a fixed distance.

In the first embodiment described above, the range of the region to be magnified (from the reference position to the edge of the region to be magnified) is determined by the control unit 113 and the reduction scale factor and range of the region to be reduced (from the edge of the region to be magnified in the radial direction to the edge of the region to be reduced also in the radial direction) on the basis of the magnification scale factor and range of the region to be magnified. The present technology is not limited thereto, the scale factor and range of the region to be magnified may be arbitrarily determined.

Referring to the upper graph of Fig. 6, for example, in a case where the distance B at which the edge of the region to be reduced is located is set as a fixed value, the length from the reference position, which is the range of the region to be magnified, to the distance D is set to be longer. That is, the length from the distance D to the distance B is shortened.

In this case, the reduced region becomes narrower, and therefore the target image displayed in the region to be reduced is further reduced. Moreover, the target image is displayed at the same scale factor at the distance B at which the edge of the region to be reduced is located.

That is, on the basis of the range and the scale factor of the region to be magnified set by the user, the magnified and reduced image is generated such that the same-scale factor region (solid line 53) is set such that the positional relationship between the target image and the magnified and reduced image becomes the same within the range from the distance D to the distance C (from the reduced region to the image edge of the magnified and reduced image).

Cooperation of a computer installed in the communication terminal and another computer capable of communicating therewith via a network or the like may perform the information processing method and the program according to the present technology and may construct the information processing device according to the present technology.

That is, the information processing method and the program according to the present technology can be executed not only in a computer system configured by a single computer but also in a computer system in which a plurality of computers cooperate each other. It should be noted that in the present disclosure, the system means a set of components (such as devices and modules (parts)) and it does not matter whether all of the components are in a single casing. Therefore, a plurality of devices housed in separate casings and connected to one another via a network and a single device having a plurality of modules housed in a single casing are both the system.

Execution of the information processing method and the program according to the present technology by the computer system includes, for example, both a case where the image recognition, the determination of the surgical technique, and the generation of the magnified and reduced image, and the like are performed by a single computer and a case where the respective processes are performed by different computers. Moreover, performing the respective processes by a predetermined computer includes causing another computer to perform some or all of those processes and obtaining results thereof.

That is, the information processing method and the program according to the present technology can also be applied to a cloud computing configuration in which a single function is shared and commonly processed by a plurality of devices via a network.

The respective configurations such as the image information acquisition unit, the image recognition unit, the presentation image generation unit, and the control unit, which have been described above with reference to the drawings are merely one embodiment, and can be arbitrarily modified without departing from the gist of the present technology. That is, any other configurations, algorithms, and the like for carrying out the present technology may be employed.

It should be noted that the effects described in the present disclosure are merely illustrative, not limitative, and other effects may be provided. The above description of the plurality of effects does not mean that those effects are always provided at the same time. It means that at least any of the above-mentioned effects can be obtained in a manner that depends on conditions and the like and effects not described in the present disclosure can be provided as a matter of course.

At least two of the features in the respective embodiments described above may be combined. In other words, various features described in the respective embodiments may be arbitrarily combined across the embodiments. Moreover, the various effects described above are merely illustrative, not limitative, and other effects may be provided.

It should be noted that the present technology can also take the following configurations.
(1) A surgical microscope system, including:
   a microscope optical system;
   an imaging device that captures an image of a visual field range including an eye to be treated through the microscope optical system; and
   an image processing device that magnifies a first region in an image including the eye to be treated based on the image captured by the imaging device and reduces a second region other than the first region in the image to be capable of being displayed in a region other than a magnified region that is the first region magnified in the image.
(2) The surgical microscope system according to (1), in which
   the image is at least a part of a captured image obtained by imaging the eye to be treated.
(3) The surgical microscope system according to (1) or (2), in which
   the image processing device sets a peripheral region adjacent to a periphery of the first region and a region other than the peripheral region to the second region and reduces the peripheral region.
(4) The surgical microscope system according to any one of (1) to (3), in which
   the image processing device acquires treatment information including at least one of information regarding the eye to be treated or information regarding a treatment to be performed on the eye as a target and determines the first region on the basis of the treatment information.
(5) The surgical microscope system according to (4), in which
   the image processing device acquires the treatment information on the basis of the image.
(6) The surgical microscope system according to (4) or (5), in which
   the image processing device acquires the treatment information from outside.
(7) The surgical microscope system according to any one of (4) to (6), in which
   the treatment information includes at least one of details of the treatment, an instrument to be used in the treatment, a range to be illuminated, a site of the eye, a lesion, or an outline of the image.
(8) The surgical microscope system according to any one of (1) to (7), in which
   the image processing device
   is configured to be capable of receiving an instruction of a user, and
   is configured to determine the first region on the basis of the instruction of the user.
(9) The surgical microscope system according to any one of (1) to (8), in which
   the image processing device sets a predetermined position in the first region as a reference position and magnifies the first region by using the reference position as a reference.
(10) The surgical microscope system according to (9), in which
   the image processing device magnifies the first region such that a distance between the reference position and each position of the first region is increased.
(11) The surgical microscope system according to (9) or (10), in which
   the image processing device reduces the second region by using the reference position as the reference.
(12) The surgical microscope system according to (9) to (11), in which
   the image processing device reduces the second region such that a distance between the reference position and each position of an outermost portion of the image.
(13) The surgical microscope system according to (9) to (12), in which
   the image processing device sets a peripheral region adjacent to a periphery of the first region and a region other than the peripheral region to the second region and reduces the second region such that a distance between the reference position and each position of the region other than the peripheral region is maintained.
(14) The surgical microscope system according to (9) to (13), in which
   the image processing device acquires treatment information including at least one of information regarding an eye to be treated or information regarding a treatment to be performed on the eye as a target and determines the reference position on the basis of the treatment information.
(15) The surgical microscope system according to (9) to (14), in which
   the image processing device
   is configured to be capable of receiving an instruction of a user, and
   is configured to determine the reference position on the basis of the instruction of the user.
(16) The surgical microscope system according to (1) to (15), in which
   the image processing device is configured to be capable of generating an auxiliary image representing a state of magnification with respect to the first region and representing a state of reduction with respect to the second region.
(17) The surgical microscope system according to (16), in which
   the auxiliary image is an image in which the state of magnification and the state of reduction are represented by using at least one of a grid pattern, a luminance, a saturation, a hue, or a boundary line.
(18) An image processing method, including:
   by a computer system,
   acquiring an image including an eye to be treated; and
   magnifying a first region in the image and reducing a second region other than the first region in the image to be capable of being displayed in a region other than a magnified region that is the first region magnified in the image.
(19) A program that causes a computer system to execute:
   a step of acquiring an image including an eye to be treated; and
   a step of magnifying a first region in the image and reducing a second region other than the first region in the image to be capable of being displayed in a region other than a magnified region that is the first region magnified in the image.
(20) An image processing device, including:
   an image acquisition unit that acquires an image including an eye to be treated; and
   an image processing unit that magnifies a first region in the image and reduces a second region other than the first region in the image to be capable of being displayed in a region other than a magnified region that is the first region magnified in the image. Reference Signs List

- 10: target image
- 11: region to be magnified
- 12: region to be reduced
- 20: magnified and reduced image
- 21: magnified region
- 22: reduced region
- 35: reference position
- 100: surgical microscope system
- 102: microscope
- 104: image processing device
- 108: user input unit
- 109: instrument
- 110: image information acquisition unit
- 111: image recognition unit
- 112: presentation image generation unit
- 113: control unit
- 114: interface unit
- 137: auxiliary image

## Claims

1. A surgical microscope system, comprising:
a microscope optical system;
an imaging device that captures an image of a visual field range including an eye to be treated through the microscope optical system; and
an image processing device that magnifies a first region in an image including the eye to be treated based on the image captured by the imaging device and reduces a second region other than the first region in the image to be capable of being displayed in a region other than a magnified region that is the first region magnified in the image.

2. The surgical microscope system according to claim 1, wherein
the image is at least a part of a captured image obtained by imaging the eye to be treated.

3. The surgical microscope system according to claim 1, wherein
the image processing device sets a peripheral region adjacent to a periphery of the first region and a region other than the peripheral region to the second region and reduces the peripheral region.

4. The surgical microscope system according to claim 1, wherein
the image processing device acquires treatment information including at least one of information regarding the eye to be treated or information regarding a treatment to be performed on the eye as a target and determines the first region on a basis of the treatment information.

5. The surgical microscope system according to claim 4, wherein
the image processing device acquires the treatment information on a basis of the image.

6. The surgical microscope system according to claim 4, wherein
the image processing device acquires the treatment information from outside.

7. The surgical microscope system according to claim 4, wherein
the treatment information includes at least one of details of the treatment, an instrument to be used in the treatment, a range to be illuminated, a site of the eye, a lesion, or an outline of the image.

8. The surgical microscope system according to claim 1, wherein
the image processing device
is configured to be capable of receiving an instruction of a user, and
is configured to determine the first region on a basis of the instruction of the user.

9. The surgical microscope system according to claim 1, wherein
the image processing device sets a predetermined position in the first region as a reference position and magnifies the first region by using the reference position as a reference.

10. The surgical microscope system according to claim 9, wherein
the image processing device magnifies the first region such that a distance between the reference position and each position of the first region is increased.

11. The surgical microscope system according to claim 9, wherein
the image processing device reduces the second region by using the reference position as the reference.

12. The surgical microscope system according to claim 9, wherein
the image processing device reduces the second region such that a distance between the reference position and each position of an outermost portion of the image.

13. The surgical microscope system according to claim 9, wherein
the image processing device sets a peripheral region adjacent to a periphery of the first region and
a region other than the peripheral region to the second region and reduces the second region such that a distance between the reference position and each position of the region other than the peripheral region is maintained.

14. The surgical microscope system according to claim 9, wherein
the image processing device acquires treatment information including at least one of information regarding an eye to be treated or information regarding a treatment to be performed on the eye as a target and determines the reference position on a basis of the treatment information.

15. The surgical microscope system according to claim 9, wherein
the image processing device
is configured to be capable of receiving an instruction of a user, and
is configured to determine the reference position on a basis of the instruction of the user.

16. The surgical microscope system according to claim 1, wherein
the image processing device is configured to be capable of generating an auxiliary image representing a state of magnification with respect to the first region and representing a state of reduction with respect to the second region.

17. The surgical microscope system according to claim 16, wherein
the auxiliary image is an image in which the state of magnification and the state of reduction are represented by using at least one of a grid pattern, a luminance, a saturation, a hue, or a boundary line.

18. An image processing method, comprising:
by a computer system,
acquiring an image including an eye to be treated; and
magnifying a first region in the image and reducing a second region other than the first region in the image to be capable of being displayed in a region other than a magnified region that is the first region magnified in the image.

19. A program that causes a computer system to execute:
a step of acquiring an image including an eye to be treated; and
a step of magnifying a first region in the image and reducing a second region other than the first region in the image to be capable of being displayed in a region other than a magnified region that is the first region magnified in the image.

20. An image processing device, comprising:
an image acquisition unit that acquires an image including an eye to be treated; and
an image processing unit that magnifies a first region in the image and reduces a second region other than the first region in the image to be capable of being displayed in a region other than a magnified region that is the first region magnified in the image.
